**Europäisches Patentamt**

**European Patent Office**

**Office européen des brevets**

(19)

(11) Publication number : **0 314 325 B1**

(12)

# EUROPEAN PATENT SPECIFICATION

(45) Date of publication of patent specification :
**18.03.92 Bulletin 92/12**

(51) Int. Cl.⁵ : **G01F 1/704, A61B 5/08**

(21) Application number : **88309385.8**

(22) Date of filing : **07.10.88**

(54) **Gas flow meter for clinical use.**

(30) Priority : **08.10.87 GB 8723623**

(43) Date of publication of application :
**03.05.89 Bulletin 89/18**

(45) Publication of the grant of the patent :
**18.03.92 Bulletin 92/12**

(84) Designated Contracting States :
**DE FR GB IT NL**

(56) References cited :
**EP-A- 0 024 327**
**US-A- 3 687 130**
**US-A- 3 962 917**
**US-A- 4 237 730**
**US-A- 4 483 200**

(73) Proprietor : **NATIONAL RESEARCH DEVELOPMENT CORPORATION**
**101 Newington Causeway**
**London SE1 6BU (GB)**

(72) Inventor : **Hall, Peter Ronald Bryngwyddil**
**Boneffosfelen Nr. Carmarthen**
**Dyfed Wales (GB)**
Inventor : **Noori, Amir Hassan Malek Mohammady**
**9 Bryngoleu Terrace Sketty**
**Swansea Wales (GB)**

(74) Representative : **Froud, Clive et al**
**Elkington and Fife Prospect House 8 Pembroke Road**
**Sevenoaks, Kent TN13 1XR (GB)**

EP 0 314 325 B1

## Description

This invention relates to a gas flow meter for clinical use; more particularly, it relates to a pulsed wire fluid flow monitoring device or a tidal volume monitoring device.

Pneumotachometers or anemometers are instruments that measure the instantaneous flow rate of respired gas. In a clinical environment, they are often used to measure forced expiratory flow, maximum mid-expiratory flow and peak flow rates (see, for example, Plaut, D.I., and Webster, J.G., "Ultrasonic measurement of respiratory flow", IEEE Trans. on BME, 27, 10). Although it is only the flow rate that they may measure directly, absolute volume changes may be obtained by integrating the flow rate. By combining the flow rate and volume, the 'flow volume loop' may also be obtained which may be a great help to a physician in obtaining information about the lung and diagnosing diseases, such as emphysema and asthma (see, for example, Plaut and Webster, loc cit; Polo, W.M., "Pneumotachography", Acta Anesth. Scand., Suppl. XI, 171-178; Buess, C., et al, "Design and construction of a pulsed ultrasonic air flowmeter", IEEE Trans. on BME, 33, 8; Hyatt, R.E., and Black, L.F. , "The flow-volume curve a current perspective", American Review of Respiratory Disease, 107).

For monitoring Neonates, it is generally accepted that a dynamic range of from 0 to 100 ml/s, a dead space of less than 1.8 ml and a frequency response of 50 Hz or more are adequate. For many applications, it is also necessary to know the direction of flow (i.e. inhalation or exhalation). Some instruments are unable to tell this and they are fitted with one-way valves to differentiate between inhalation and exhalation. There are many different types of pneumotachographs each having certain advantages and disadvantages, the ones most commonly used in clinical environments being Fleisch Pneumotachographs, Wright's Respirometers and Hot wire anemometers.

Fleisch Pneumotachographs comprise a bundle of narrow tubes placed in the path of the gas flow. As the gas passes through these tubes, a pressure drop, which is linearly related to the flow rate, is developed across them. this pressure drop is measured by a differential pressure transducer calibrated to read out directly in units of flow rate. The main disadvantage is in the requirement that the flow must be laminar for the relation between the flow rate and the pressure drop to be linear. Consequently, there are eight different sizes of flow head to accommodate flow rates up to 14 litres/min. Over prolonged use, the sensor becomes clogged with mucus which changes the flow pattern and therefore the accuracy. Another disadvantage of the Fleisch Pneumotachograph is its viscosity-dependency (see, for example, Plant and Webster, loc cit; Hyatt and Black, loc cit; Sullivan, W.J., et al, "Pneumatachographs: Theory and clinical application", Respiratory Care, 29, 7).

Wright's Respirometer measures the flow by placing a turbine whose rotation rate is related to flow rate in the respiratory path. It suffers from errors caused by mechanical inertia and friction (see, for example, Plant and Wester, loc cit).

Hot wire anemometry is based on the principle that the passage of cool air over a substance tends to reduce the temperature of that substance by an amount depending on the flow rate (see, for example, Sullivan, et al, loc cit). Instruments based on this principle usually employ a short tungsten wire heated to a constant temperature well above the temperature of the air in the lungs, say at 50 degrees centigrade. Respiratory air flows over the wire and, at higher flow rates, more electrical energy is needed to keep the wire temperature constant. This energy is not linearly related to the flow rate, but follows a complex relation to, inter alia the ambient temperature, gas viscosity and specific heat capacity. This necessitates complex linearising to counteract the above shortcomings. These linearisers are usually effective at only one flow rate. Furthermore, these sensors are generally inherently unidirectional which makes them impractical for measuring tidal volumes. A possible modification to such a sensor is to use one-way valve to add bidirectionality. This is a mechanical device and leads to a reduced frequency response.

Although certain aspects of the apparatus now provided are known individually, the present invention has been neither taught nor suggested by the prior art. For example: US-A-3,962,917 discloses one hot wire between two sensor wires, for flow direction determination, with a second hot wire, which is not for direction discrimination; US-A-4,483,200 shows two hot wires and two sensor wires downstream thereof mounted in the same plane perpendicular to the flow; and US-A-4,237,730 describes an upstream hot wire and downstream two sensors in the same plane perpendicular to the flow.

More particularly, the present invention provides a pulsed hot wire anemometer apparatus comprising: a longitudinal conduit, two heated wires (10,11) and two temperature-sensitive sensor wires (12,13) in the conduit, the sensor wires being located spaced apart in a plane perpendicular to the axis of the conduit, one (10) of the said heated wires and one (12) of the said sensor wires being in a first plane parallel to the axis of the conduit, and the other (11) of the said heated wires and the other (13) of the said sensor wires being in a second plane parallel to the axis of the conduit, characterised in that the heated wires are positioned respectively on different sides of, and spaced from, the said sensor wire plane, the arrangement being such that, in one direction of flow of fluid through the conduit, the said one heated wire (10) influences the said one sensor wire (12), with the said other sensor wire (13) acting as a reference sensor, and, in the opposite direction of flow, the said other heated wire (11) influences the said other sensor wire (13), with the said one sensor wire (12) acting as a reference sensor.

Among the advantages of the present invention are that it is independent of gas composition and ambient temperature, it has no moving parts and hence fast response time, it has a high dynamic range of flow rate, it presents low resistance to the respiratory path and it is very light and compact with low dead space.

The present invention is exemplified with reference to the following drawings:

Figure 1 is an illustrative diagram of the electronic circuitry in the information processing steps;

Figure 2 is an illustrative plan view of the sensor head in Figure 1;

Figure 3 is an illustrative graph of signals in the transmitting and receiving wires;

Figure 4 illustrates the leakage occurring in the present device;

Figure 5 is an illustrative graph of signal versus time in the pulsed wire and in the sensor wire.

Figure 6 illustrates a typical flow waveform with no leak;

Figure 7 illustrates a typical flow waveform in the presence of a leak.

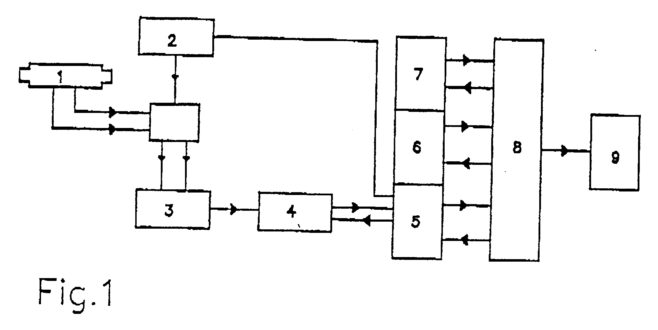

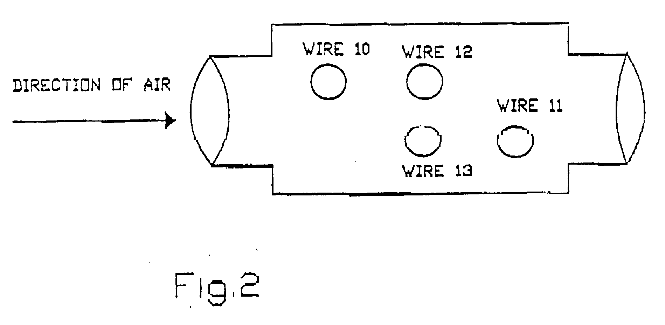

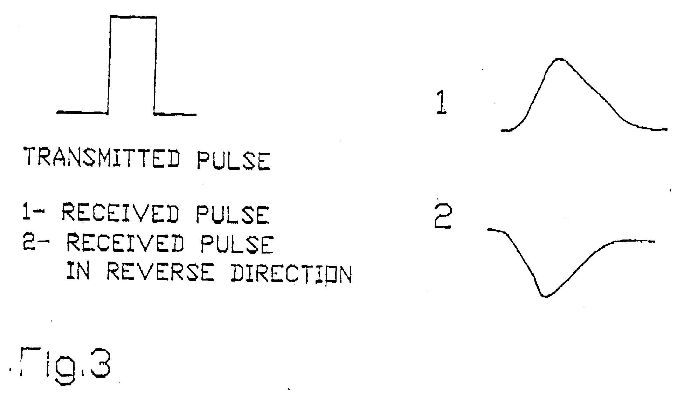

In accompanying Figure 1, the details of the circuitry necessary to process the information from the sensor head are illustrated. Referring also to accompanying Figure 2, the sensor head (1) comprises two transmitting wires (10) and (11), one sensor wire (12) and a reference wire (13), receiving wires. A pulse of, for example, 75 μs duration is applied to transmitting wires (10) and (11) by pulse generator (2). A cylinder of heat is formed around these wires. It is carried to either left or right depending on the respiratory cycle. When the flow is in the direction shown in Figure 2, (10) is the transmitting wire, (12) is the receiving wire and (13) is the reference wire. Wires (12) and (13) form two arms of a Wheatstone bridge. The receiving wire will be hotter than the reference wire (13) (since it is placed directly in front of the transmitting wire) and so its resistance will change by a higher proportion. In the other direction, the roles of the wires are interchanged and so the received pulse is inverted (see accompanying Figure 3).

The receiving and reference wires are in the same plane perpendicular to the air flow, so the resistance thereof changes by the same amount if the bulk temperature of the gas changes. This makes the bridge output independent of the above parameters.

A smooth plate is preferably placed between the receiving wires parallel to the direction of the flow so that the receiving wires will be in the boundary layer of the plate where the flow will be more laminar. With this embodiment, the range over which the flow is laminar is increased. There are generally also meshes at either end of the sensor to make the flow pattern inside the sensor independent of the geometry of the connecting tubing, which also produces a more even flow.

When the transmitting pulse is initiated, the transmitting wires reach a temperature of about 500 degrees centigrade. In very humid conditions, water condenses on the transmitting wire when the wires are cold (i.e. the pulse is not being sent). As the transmitting pulse is being sent, water droplets tend to explode and take away some part of the metal and eventually cause the breakage of the transmitting wires. Generally, a D.C. current is passed through each transmitting wire to keep its temperature at about 70 degrees centigrade so that no droplets form on the transmitting wire. In addition, the whole sensor is generally heated to about 45 degrees centigrade to prevent water from condensing inside the sensor and changing the flow pattern.

Two calibration potentiometers are preferably incorporated in the sensor to calibrate the flow rate in either direction.

The transmitting and the receiving wires have to be very tight to maintain response to high flow rate. To make the task of placing the wires easier, one end of the wire is preferably fixed to a spring that helps to keep the wire tight. The wires may be glued in afterwards to prevent their movement.

The instrument itself contains a differential amplifier whose output is proportional to the temperature difference between the two receiving wires. The output of this amplifier feeds a comparator system. The crucial point is that the comparator measuring the resistance rise in the sensor wire is set to its reference level just before the resistance rise is expected, since this level is dependent on the temperature of the respired gas and a large temperature difference exists between inspired and respired gases. Just before the received pulse is expected, the baseline is sampled by a sample-and-hold circuit and presented to the comparator circuitry, this being an aspect of microcomputer control. The comparator continually compares the received signal against this reference level and, if the received pulse is over a certain positive threshold or under a certain negative threshold, the comparator concludes that the pulse has arrived in either positive or negative direction and stops the timer T2 which reflects the time of flight.

Further circuitry (5) is associated with the interface electronics.

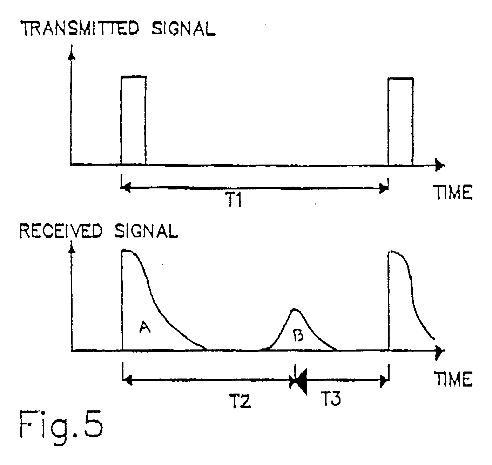

Accompanying Figure 5 depicts graphically the pulsed wire signal and the corresponding detected signals in the sensor wire. Due to electromagnetic induction in the sensor wire from either of the pulsed wires, the received signal is composed of an unwanted induced signal (A) and a signal (B) which results from the pulse of heat arriving at the sensor wire.

There are two timing devices, one device (7) measures the actual time ($t_1$) between successive pulses being sent, the second device (6) measures the time ($t_2$) between sending a pulse and the detection of the pulse at the sensor wire. As in accompanying Figure 5, there is a delay time ($t_3$) to allow for a series of calculations, manipulations and display routines which are performed before the next sample may be initiated. Thus, the time between samples ($t_1$) is a variable figure equal to the flight time, where flight time is the time taken for the electrically generated heat pulse to reach the sensor wire i.e. $t_2$, plus an amount of programming time ($t_3$). Therefore, the only accurate way of assessing volume flow is to apply the flow rate ($1/t_2$) obtained in a sample over the time $t_1$ between initiation of successive samples.

The results are generally displayed on a display unit (9) which gives details of:-
flow rate = $1/t_2$ x constant, flow volume per sample = $t_1$ x $1/t_2$ x constant, accumulative volume (since last direction change, previous instroke and outstroke volumes) and tidal volume = mean of instroke and outstroke, respiratory rate, minute volume.

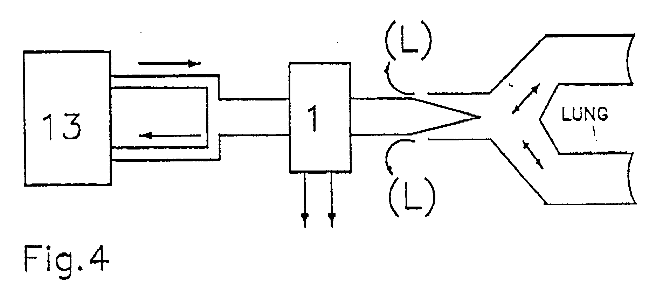

As illustrated in accompanying Figure 4, the present instrument may be used to estimate leaks, i.e. the difference between inspiration and expiration volume.

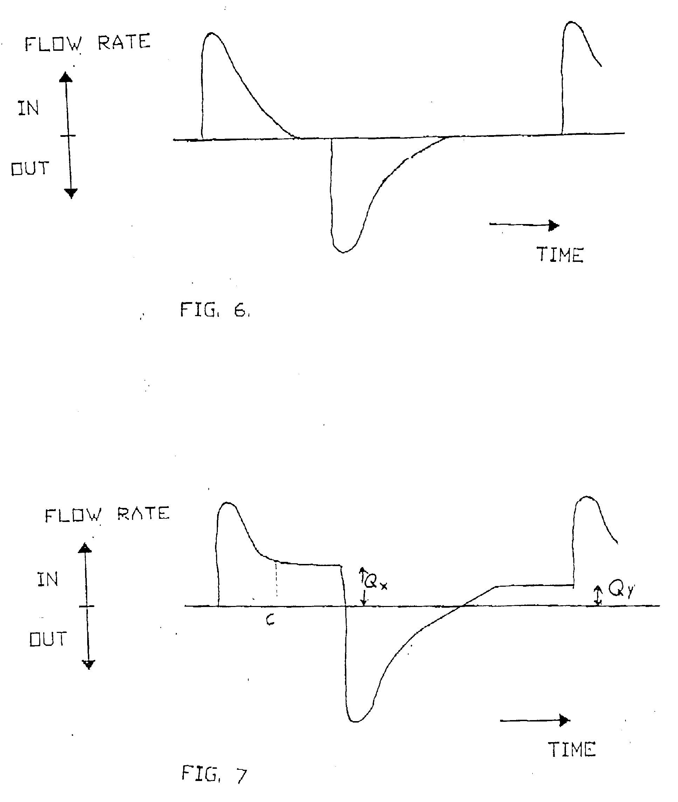

Leakage may be estimated as follows:
The ventilator is set to a low respiration rate of the order of 10 breaths per minute so that the flow rate reaches a steady value during each respiration cycle. If there is no leak, the flow waveform approximates that illustrated in accompanying Figure 6. In the presence of a leak, the flow pattern changes to such as illustrated in accompanying Figure 7. At point C, the pressure inside the lungs and the ventilator are equal and the flow into the lungs drops to zero and Qx is the leakage flow rate during inspiration time. The same applies during expiration, i.e. Qy is the leakage flow rate during expiration (if PFFP is not applied then Qy drops to zero). Assuming the leak flow rate is not changed during the whole inspiration and expiration cycle (which is true to within a few percent at low respiration rates) the volume leak during inspiration may be calculated as Linspiration = Qx * inspiration time, while the volume leak during expiration is calculated as Lexpiration = Qy * expiration time. The Alveolar tidal volume (true tidal volume) is measured as either Alveolar tidal volume1 = measured inspiration volume - Linspiration or Alveolar tidal volume2 = measured expiration volume + Lexpiration. Errors may be reduced by taking the average of the above two values hence:

Alveolar tidal volume = (alveolar tidal volume 1 + alveolar tidal volume 2) / 2. ADDITIONALLY:
1 - leak volume during each breath is Linspiration + Lexpiration.
2 - (Linspiration + Lexpiration) * respiratory rate is equal to minute leak.

At the point the gas flow changes direction, some glitches may occur on the calculated flow rate. This is because the change in the gas flow direction feeds the hot cylinders of gas back over the receiving wires a second time. The effect is a very short registered flight time or a very high flow rate. To counteract the above problem, a deglitching algorithm is implemented in software. The software continually holds three samples (sample 1 = present sample; sample 2 = previous sample; sample 3 = sample before previous sample). If sample 2 is significantly greater in size than either sample 1 or sample 3, and the flow is on the point of reversing or has just reversed its direction, then sample 2 is replaced by sample 3 or sample 1 depending on the direction of the flow.

**Claims**

1. A pulsed hot wire anemometer apparatus comprising: a longitudinal conduit, two heated wires (10,11) and two temperature-sensitive sensor wires (12,13) in the conduit, the sensor wires being located spaced apart in a plane perpendicular to the axis of the conduit, one (10) of the said heated wires and one (12) of the said sensor wires being in a first plane parallel to the axis of the conduit, and the other (11) of the said heated wires and the other (13) of the said sensor wires being in a second plane parallel to the axis of the conduit, characterised in that the heated wires are positioned respectively on different sides of, and spaced from, the said sensor wire plane, the arrangement being such that, in one direction of flow of fluid through the conduit, the said one heated wire (10) influences the said one sensor wire (12), with the said other sensor wire (13) acting as a reference sensor, and, in the opposite direction of flow, the said other heated wire (11) influences the said other sensor wire (13), with the said one sensor wire (12) acting as a reference sensor.

2. An apparatus as claimed in claim 1 wherein a smooth plate is positioned between the sensor wires in a plane parallel to gas flow.

3. An apparatus as claimed in claim 1 or claim 2 wherein the heated wires are provided with condensation-preventing DC heating means.

4. An apparatus as claimed in any of claims 1 to 3 wherein the heated and sensor wires are positioned in a sensor head, which may be provided with condensation-preventing heating means.

5. An apparatus as claimed in any of claims 1 to 4 wherein the heated and sensor wires are provided with tensioning spring means.

6. An apparatus as claimed in any of claims 1 to 5 wherein means are provided to sample the baseline temperature immediately prior to expected arrival of a transmitted heat pulse.

7. An apparatus as claimed in any of claims 1 to 6 wherein means are provided to sample the flow rate immediately before the inspiration or expiration cycle starts to estimate leaks.

8. The use of an apparatus as claimed in any of claims 1 to 7 to measure respiration.

**Patentansprüche**

1. Impulsbetriebene Hitzedrahtanemometereinrichtung, umfassend: einen längsverlaufenden Kanal, zwei geheizte Drähte (10, 11) und zwei temperaturempfindliche Sensordrähte (12, 13) in dem Kanal, wobei die Sensordrähte in einer zu der Achse des Kanals senkrechten Ebene im bestand voneinander angeordnet sind, wobei einer (10) der geheizten Drähte und einer (12) der Sensordrähte in einer ersten Ebene ist, die parallel zu der Achse des Kanals ist, und wobei der andere (11) der geheizten Drähte und der andere (13) der Sensordrähte in einer zweiten Ebene ist, die parallel zu der Achse des Kanals ist, dadurch **gekennzeichnet** , daß die geheizten Drähte jeweils auf unterschiedlichen Seiten und im Abstand von der Sensordrahtebene positioniert sind, wobei die Anordnung derart ist, daß in einer Richtung der Strömung von Fluid durch den Kanal der eine geheizte Draht (10) den einen Sensordraht (12) beeinflußt, wobei der andere Sensordraht (13) als ein Bezugssensor funktioniert, und in der entgegengesetzten Richtung der Strömung der andere geheizte Draht (11) den anderen Sensordraht (13) beeinflußt, wobei der eine Sensordraht (12) als ein Bezugssensor funktioniert.

2. Einrichtung nach Anspruch 1, worin eine glatte Platte zwischen den Sensordrähten in einer Ebene parallel zur Gasströmung positioniert ist.

3. Einrichtung nach Anspruch 1 oder Anspruch 2, worin die geheizten Drähte mit einer kondensationsverhindernden Gleichstromheizeinrichtung versehen sind.

4. Einrichtung nach irgendeinem der Ansprüche 1 bis 3, worin die geheizten Drähte und die Sensordrähte in einem Sensorkopf positioniert sind, der mit einer kondensationsverhindernden Heizeinrichtung versehen sein kann.

5. Einrichtung nach irgendeinem der Ansprüche 1 bis 4, worin die geheizten Drähte und die Sensordrähte mit einer spannenden Federeinrichtung versehen sind.

6. Einrichtung nach irgendeinem der Ansprüche 1 bis 5, worin Mittel zum Abtasten der Basislinientemperatur unmittelbar vor der erwarteten Ankunft eines übertragenen Erhitzungsimpulses vorgesehen sind.

7. Einrichtung nach irgendeinem der Ansprüche 1 bis 6, worin Mittel zum Abtasten der Strömungsrate unmittelbar vor dem Beginn des Einatmungs- oder Ausatmungszyklus zum Beurteilen von Lecks vorgesehen sind.

8. Verwendung der Einrichtung nach irgendeinem der Ansprüche 1 bis 7 zum Messen der Atmung.

**Revendications**

1. Appareil anémométrique pulsé à fil chaud, comprenant : un conduit longitudinal, deux fils chauffés (10, 11) et deux fils capteurs (12, 13) sensibles à la température et placés dans le conduit, les fils capteurs étant séparés dans un plan perpendiculaire à l'axe du conduit, l'un (10) des fils chauffés et l'un (12) des fils capteurs se trouvant dans un premier plan parallèle à l'axe du conduit, et l'autre (11) des fils chauffés et l'autre (13) des fils capteurs se trouvant dans un second plan parallèle à l'axe du conduit, caractérisé en ce que les fils chauffés sont placés respectivement sur des côtés différents du plan des fils capteurs et sont distants de ce plan, la disposition étant telle que, dans un premier sens de circulation du fluide dans le conduit, le premier fil chauffé (10) influence le premier fil capteur (12), alors que l'autre fil capteur (13) joue le rôle d'un capteur de référence et, dans l'autre sens de circulation, l'autre fil chauffé (11) influence l'autre fil capteur (13), le premier fil capteur (12) jouant le rôle d'un capteur de référence.

2. Appareil selon la revendication 1, dans lequel une plaque lisse est placée entre les fils capteurs dans un plan parallèle au débit de gaz.

3. Appareil selon la revendication 1 ou 2, dans lequel les fils chauffés comportent un dispositif de chauffage en courant continu destiné à empêcher la condensation.

4. Appareil selon l'une quelconque des revendications 1 à 3, dans lequel les fils chauffés et capteurs sont placés dans une tête capteur qui peut comporter un dispositif de chauffage destiné à empêcher la condensation.

5. Appareil selon l'une quelconque des revendications 1 à 4, dans lequel les fils chauffés et capteurs sont munis d'un dispositif à ressort de tension.

6. Appareil selon l'une quelconque des revendications 1 à 5, dans lequel un dispositif est destiné à échantillonner la température de référence juste avant l'arrivée prévue d'une impulsion émise de chaleur.

7. Appareil selon l'une quelconque des revendications 1 à 6, dans lequel un dispositif est destiné à échantillonner le débit juste avant le début du cycle d'aspiration ou d'expiration afin que les fuites soient estimées.

8. Application de l'appareil selon l'une quelconque des revendications 1 à 7 à la mesure de la respiration.

Fig.1

DIRECTION OF AIR

WIRE 10    WIRE 12

WIRE 11

WIRE 13

Fig.2

TRANSMITTED PULSE

1- RECEIVED PULSE
2- RECEIVED PULSE
    IN REVERSE DIRECTION

1

2

Fig.3

Fig.4

TRANSMITTED SIGNAL

TIME

T1

RECEIVED SIGNAL

A

B

TIME

T2    T3

Fig.5

FLOW RATE

IN

OUT

TIME

FIG. 6.

FLOW RATE

IN

OUT

$Q_x$

$c$

$Q_y$

TIME

FIG. 7